(19) Europäisches Patentamt
European Patent Office
Office européen des brevets

(11) Veröffentlichungsnummer: **0 113 044**
**B1**

(12) **EUROPÄISCHE PATENTSCHRIFT**

(45) Veröffentlichungstag der Patentschrift: **03.09.86**

(51) Int. Cl.⁴: **C 07 C 119/042,** C 07 C 118/02, C 08 G 18/74

(21) Anmeldenummer: **83111853.4**

(22) Anmeldetag: **26.11.83**

(54) **Diisocyanate oder Diisocyanat-Gemische, ein Verfahren zu ihrer Herstellung, sowie ihre Verwendung zur Herstellung von Polyurethankunststoffen.**

(30) Priorität: **08.12.82 DE 3245321**

(43) Veröffentlichungstag der Anmeldung: **11.07.84 Patentblatt 84/28**

(45) Bekanntmachung des Hinweises auf die Patenterteilung: **03.09.86 Patentblatt 86/36**

(84) Benannte Vertragsstaaten: **BE DE FR GB IT**

(56) Entgegenhaltungen: **US-A-3 663 514**

(73) Patentinhaber: **BAYER AG, Konzernverwaltung RP Patentabteilung, D-5090 Leverkusen 1 Bayerwerk (DE)**

(72) Erfinder: **Knöfel, Hartmut, Dr., Dülmener Weg 21, D-5068 Odenthal (DE)**
Erfinder: **Brockelt, Michael, Neuenhauser Weg 1, D-5060 Bergisch- Gladbach 2 (DE)**
Erfinder: **Penninger, Stefan, Dr., Pommernallee 5, D-4047 Dormagen 1 (DE)**
Erfinder: **Stutz, Herbert, Dr., Schulstrasse 81, D-4047 Dormagen 5 (DE)**

**EP 0 113 044 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue, am aromatischen Ring monosubstituierte, gegebenenfalls als Isomerengemische vorliegende Isocyanatobenzyl-cyclohexylisocyanate, ein Verfahren zu ihrer Herstellung durch Phosgenierung der ihnen zugrundeliegenden Diamine bzw. Diamingemische, sowie ihre Verwendung als Aufbaukomponente bei der Herstellung von Polyurethankunststoffen.

Asymmetrische Diisocyanate, welche eine aromatisch und eine cycloaliphatisch gebundene Isocyanatgruppe aufweisen eignen sich wegen der stark unterschiedlichen Reaktivität dieser Isocyanatgruppen ausgezeichnet zur Herstellung von Polyurethankunststoffen nach dem Prepolymerverfahren, wobei zunächst die höherreaktive, aromatisch gebundene Isocyanatgruppe unter Bildung des NCO-Prepolymeren abreagiert, welches dann in einer zweiten Reaktionsstufe zum hochmolekularen Polyurethan umgesetzt wird, oder zur Herstellung von modifizierten Diisocyanaten, beispielsweise von Uretdiondiisocyanaten, wobei auch hier in einer ersten Reaktionsstufe die höherreaktive Isocyanatgruppe unter Dimerisierung abreagiert, so daß ein Uretdiongruppen aufweisendes Diisocyanat erhalten wird, welches mit Verbindungen gegenüber Isocyanatgruppen reaktionsfähigen Gruppen weiter umgesetzt werden kann. Die US-PS 3 663 514 beschreibt nun bereits ein derartiges Diisocyanat, nämlich das 4-(4-Isocyanato-benzyl)-cyclohexylisocyanat, jedoch scheiterte bislang ein großtechnischer Einsatz dieses Diisocyanats an dem Umstand, daß das, dem Diisocyanat zugrundeliegende, Diamin durch asymmetrische Kernhydrierung nur in Ausbeuten von weniger als 35 % der Theorie zugänglich und daher das reine asymmetrische Diisocyanat nur mit erheblichem Reinigungsaufwand zu gewinnen ist.

Es war die der vorliegenden Erfindung zugrundeliegende Aufgabe, neue Diisocyanate mit aromatisch und cycloaliphatisch gebundenen Isocyanatgruppen aufzufinden, die möglichst bei Raumtemperatur flüssig und niedrigviskos sein sollten, die bezüglich Löslichkeit und Verträglichkeit mit den, Hydroxylgruppen aufweisenden, Reaktionspartnern den Forderungen der technischen Polyurethanchemie genügen, die nur geringe Verunreinigungen an unhydrierten bzw. perhydrierten Diisocyanaten aufweisen, und deren Herstellung mit hohen Selektivitäten, d.h. in hohen Ausbeuten gelingt.

Diese Aufgabe konnte überraschenderweise durch die Bereitstellung der nachstehend näher beschriebenen Diisocyanate bzw, Diisocyanatgemische und des zu ihrer Herstellung geeigneten Verfahrens gelöst werden.

Gegenstand der Erfindung sind, gegebenenfalls Isomerengemische darstellende und gegebenenfalls im Gemisch mit untergeordneten Mengen an den entsprechenden perhydrierten Diisocyanaten und/oder im Gemisch mit untergeordneten Mengen an den entsprechenden unhydrierten aromatischen Diisocyanaten vorliegende Diisocyanate der Formel

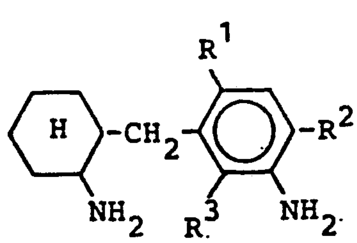

in welcher

$R^1$, $R^2$ und $R^3$ für gleiche oder verschiedene Reste stehen und Wasserstoff oder (gegebenenfalls verzweigte) Alkylgruppen mit 1 bis 12 Kohlenstoffatomen bedeuten, mit der Einschrankung, daß zwei der Reste $R^1$, $R^2$ und $R^3$ für Wasserstoff stehen und

m und n jeweils für 0 oder 1 stehen, mit der Maßgabe, daß die Summe m + n den Wert 1 ergibt, wobei im Falle von m oder n = 0 die freie Valenz durch Wasserstoff abgesättigt ist.

Gegenstand der Erfindung ist auch ein Verfahren zur Herstellung dieser Diisocyanate bzw. Diisocyanatgemische, dadurch gekennzeichnet, daß man, gegebenenfalls Isomerengemische darstellende und gegebenenfalls im Gemischmit untergeordneten Mengen an den entsprechenden perhydrierten Diaminen und/oder gegebenenfalls im Gemisch mit untergeordneten Mengen an den entsprechenden unhydrierten aromatischen Diaminen vorliegende, Diamine der Formel

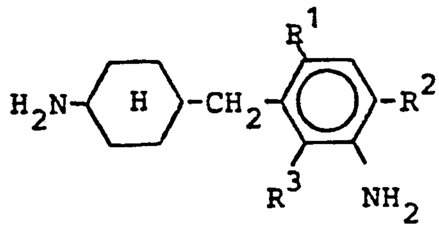

in welcher
$R^1$, $R^2$, $R^3$, m und n die obengenannte Bedeutung haben,
bei -20°C bis +250°C phosgeniert.

Gegenstand der Erfindung ist auch die Verwendung der neuen Diisocyanate bzw. Diisocyanatgemische als Aufbaukomponente bei der Herstellung von Polyurethankunststoffen nach dem Isocyanat-Polyadditionsverfahren.

Ausgangsmaterialien für das erfindungsgemäße Verfahren sind die den neuen Diisocyanaten bzw. Diisocyanatgemischen entsprechenden Diamine bzw. Diamingemische. Diese Diamine bzw. die Hauptkomponente(n) dieser Diamingemische entsprechen der zuletzt genannten allgemeinen Formel, wobei
$R^1$, $R^2$, $R^3$, m und n die bereits obengenannte Bedeutung haben,
wobei jedoch vorzugsweise einer der Reste $R^1$, $R^2$ oder $R^3$ für einen $C_1$-$C_4$-Alkylrest, insbesondere einen Methylrest, steht.

Die beim erfindungsgemäßen Verfahren zum Einsatz gelangenden Diamingemische stellen oftmals Stellungs- und/ oder Stereoisomerengemische dar, wobei die einzelnen Stellungsisomeren im wesentlichen, d.h. zu mehr als 60 Gew.-%, vorzugsweise zu mehr als 90 Gew.-% der genannten allgemeinen Formel entsprechen und zum Rest andere, am aromatischen Ring alkylsubstituierte Aminobenzyl-Cyclohexylamine darstellen. Von dieser Stellungsisomerie abgesehen, liegen die beim erfindungsgemäßen Verfahren einzusetzenden Diamine bzw. Diamingemische oftmals im Gemisch mit untergeordneten Mengen an den entsprechenden perhydrierten und/oder im Gemisch mit untergeordneten Mengen an den entsprechenden unhydrierten aromatischen Diaminen vor. Unter "untergeordneten Mengen" ist hierbei im allgemeinen ein Anteil dieser perhydrierten bzw. unhydrierten Diamine von jeweils max. 10, vorzugsweise max. 5 Gew.-%, bezogen auf Gesamtgemisch, zu verstehen. Die Hauptkomponente bzw. die Hauptkomponenten der beim erfindungsgemäßen Verfahren einzusetzenden Diamine bzw. Diamingemische stellen nach NMRspektroskopischem Befund praktisch ausschließlich asymmetrische Diamine der angegebenen Struktur dar, in welchen die Alkyl-Substituenten mit dem aromatischen Ring verknüpft sind. Die Zusammensetzung der erfindungsgemäßen Diisocyanate bzw. der erfindungsgemäßen Diisocyanatgemische entspricht der genannten Zusammensetzung der beim erfindungsgemäßen Verfahren einzusetzenden Diamine bzw. Diamingemische.

Gut als Ausgangsmaterialien für das erfindungsgemäße Verfahren geeignete Diamingemische sind beispielsweise technische Gemische von Diaminen der Formel

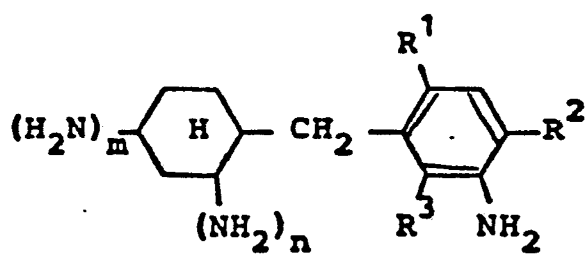

mit bis zu 40 Gew.-%, bezogen auf Gesamtgemisch an Diaminen der Formel

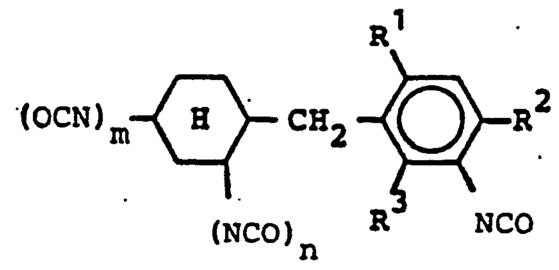

und gegebenenfalls mit bis zu 30 Gew.-%, vorzugsweise bis zu 20 Gew.-%, bezogen auf Gesamtgemisch, an anderen, am aromatischen Ring monoalkylsubstituierten Aminobenzylcyclohexylaminen, wobei in diesen technischen Gemischen untergeordnete Mengen an den entsprechenden unhydrierten und/oder perhydrierten Diaminen vorliegen können.

Besonders bevorzugt beim erfindungsgemäßen Verfahren einzusetzende Ausgangsverbindungen sind die in reiner Form vorliegenden, unter die genannte allgemeine Formel fallenden Stellungsisomeren, die die Haupt- bzw. Nebenkomponenten der genannten technischen Diamingemische darstellen. Beispiele für derartige bevorzugte Ausgangsverbindungen sind 4-(3-Amino-4-methylbenzyl)-cyclohexylamin, 2-(3-Amino-4-methylbenzyl)-cyclohexylamin, 4-(5-Amino-2-methylbenzyl)-cyclohexylamin, 2-(5-Amino-2-methylbenzyl)-cyclohexylamin, 4-(3-Amino-2-methylbenzyl)-cyclohexylamin oder 2-(3-Amino-2-methylbenzyl)-cyclohexylamin. Ebenfalls gut geeignet sind die entsprechenden Ethyl-, Propyl- oder Butyl-substituierten Aminobenzylcyclohexylamine bzw. deren Isomerengemische, die bezüglich ihrer Zusammensetzung den oben gemachten Ausführungen bezüglich der technischen Diamingemische entsprechen.

Die Herstellung der beim erfindungsgemäßen Verfahren einzusetzenden Diamine bzw. Diamingemische erfolgt durch partielle katalytische Kernhydrierung der ihnen zugrundeliegenden aromatischen Diamine oder durch zweistufige katalytische Hydrierung der ihnen zugrundeliegenden Dinitroverbindungen, welche die Vorstufe der aromatischen Diamine darstellen. Die Herstellung dieser aromatischen Vorstufen ist beispielsweise in den veröffentlichten europäischen Patentanmeldungen 0 024 665 bzw. 0 046 556 beschrieben, bzw. kann in Analogie zu den Verfahrensweisen dieser Veröffentlichungen erfolgen. So erfolgt beispielsweise die Herstellung der oben erwähnten reinen Stellungsisomeren bzw. Diamingemische, die diese Isomeren als Hauptbestandteil enthalten, durch Kondensation von p-Nitrobenzylchlorid mit p- oder o-Nitroalkylbenzolen und anschließende 2-stufige Hydrierung der aromatischen Dinitroverbindung zum teilhydrierten Diamin oder durch Kondensation von o- oder p-Nitrobenzoylchlorid mit o- oder p-Nitroalkylbenzolen, Clemmensen-Reduktion und anschließende Kernhydrierung der als Zwischenstufe anfallenden aromatischen Diamine.

Die katalytische Hydrierung der aromatischen Diamine bzw. Dinitroverbindungen wird nach den Methoden des Standes der Technik unter Anlagerung von 3 Mol Wasserstoff pro Mol Diamin bzw. Umsetzung von 9 Mol Wasserstoff pro Mol Dinitroverbindung durchgeführt. Dies bedeutet, daß die Hydrierungsreaktion vorzugsweise nach Verbrauch von 3 bzw. 9 Mol Wasserstoff pro Mol Ausgangsverbindung abgebrochen wird. Die Hydrierung erfolgt bei 20 bis 300°C unter einem Druck von 20 bis 300 bar. Im Falle der Verwendung von Dinitroverbindungen als Ausgangsmaterialien empfielt es sich, die zunächst ablaufende Hydrierung der Nitrogruppe innerhalb des Temperaturbereichs von 20 bis 150°C, vorzugsweise 30 bis 100°C, unter einem Druck von 20 bis 150 bar, vorzugsweise 70 bis 140 bar, durchzuführen und anschließend die Teilhydrierung des aromatischen Kohlenwasserstoffgerüsts bei einer Temperatur von 70 bis 300°C, vorzugsweise von 120 bis 250°C und bei einem Druck von 70 bis 300 bar, vorzugsweise 120 bis 250 bar, durchzuführen. Bei der Verwendung von aromatischen Diaminoverbindungen der beispielhaft genannten Art erfolgt die Hydrierung vorzugsweise unter den letztgenannten Bedingungen. Wie aus NMR-spektroskopischen Untersuchungen hervorgeht, bestehen die teilhydrierten "H6"-Diamine praktisch ausschließlich aus solchen Diaminen, deren Alkylsubstituenten am verbleibenden aromatischen Ring fixiert sind.

Die Hydrierungsreaktion erfolgt in Gegenwart von 0,1 bis 10 Gew.-%, vorzugsweise von 0,1 bis 1 Gew.-%, bezogen auf katalytisch wirksames Metall einerseits und Diamino- bzw. Dinitroverbindung andererseits, eines Hydrierungskatalysators. Geeignete Katalysatoren sind beispielsweise, gegebenenfalls auf inerten Trägern wie Aktivkohle, Kieselgel und insbesondere Aluminiumoxid vorliegende Elemente der 8. Nebengruppe des Periodensystems der Elemente oder katalytisch wirksame anorganische Verbindungen dieser Elemente. Besonders gut geeignet sind z.B. Ruthenium-, Platin-, Rhodium-, Nickel- und/oder Kobaltkatalysatoren in elementarer oder chemisch gebundener Form. Besonders bevorzugt werden Ruthenium oder katalytisch wirksame Rutheniumverbindungen eingesetzt. Beispiele geeigneter Rutheniumverbindungen sind Rutheniumdioxid, Rutheniumtetroxid, Bariumperruthenit, Natrium-, Kalium-, Silber-, Calcium- oder Magnesiumruthenat, Natriumperruthenat, Rutheniumpentafluorid, Rutheniumtetrafluoridhydrat oder Rutheniumtrichlorid. Falls Trägersubstanzen für die Katalysatoren mitverwendet werden, beträgt der Metallgehalt des Trägerkatalysators im allgemeinen 1 bis 10 Gew.-%, vorzugsweise 1 bis 5 Gew.-%. Im übrigen ist selbstverständlich die Art und Menge des einzusetzenden Katalysators keineswegs wesentlich, da die

Hydrierungsreaktion nach den an sich bekannten Methoden des Standes der Technik erfolgt.

Es ist oft zweckmäßig, die Hydrierungsreaktion in Gegenwart von Ammoniak durchzuführen, da Ammoniak unerwünschte Desaminierungsreaktionen und die Bildung von sekundären Aminen als Nebenprodukte unterdrückt. Falls Ammoniak mitverwendet wird, geschieht dies in Mengen von 0,1 bis 30 Gew.-%, vorzugsweise 5 bis 10 Gew.-%, bezogen auf zu hydrierende Ausgangsmaterialien.

Die Hydrierung kann lösungsmittelfrei oder in Gegenwart inerter Lösungsmittel durchgeführt werden. Im allgemeinen werden niedrigschmelzende bzw. flüssige Diamine in Substanz, hochschmelzende Diamine und Dinitroverbindungen dagegen in gelöster Form hydriert. Als Lösungsmittel eignen sich unter den Reaktionsbedingungen inerte organische Verbindungen nit niedrigem Siedepunkt, vorzugsweise Alkohole wie Methanol, Ethanol, n-Propanol, i-Propanol oder Ether wie z.B. Dioxan, Tetrahydrofuran oder Diethylether oder Kohlenwasserstoffe wie Cyclohexan. Die Hydrierung findet kontinuierlich in einem Reaktionsrohr, einer Druckkesselkaskade oder vorzugsweise diskontinuierlich in einem Rührautoklaven statt, indem man den Autoklaven mit Katalysator, der zu hydrierenden Substanz und gegebenenfalls einem Lösungsmittel beschickt, mehrmals mit Inertgas spült und gegebenenfalls Ammoniak zudosiert. Danach drückt man Wasserstoff auf, bringt das Gemisch auf Reaktionstemperatur und hydriert bis die theoretisch erforderliche Wasserstoffmenge absorbiert ist. Nach Abkühlung des Reaktionsgemischs und Abtrennung des Katalysators kann das Verfahrensprodukt gegebenenfalls destillativ von nicht-umgesetztem Ausgangsprodukt bzw. perhydriertem Diamin abgetrennt werden.

Bei der Durchführung des erfindungsgemäßen Verfahrens zur Herstellung der neuen Diisocyanate erfolgt die Phosgenierung der beispielhaft genannten Diamine oder deren Salze nach an sich bekannten Verfahren in Gegenwart eines inerten organischen Lösungsmittels (vgl. Houben-Weyl, Methoden der organischen Chemie, Georg Thieme Verlag Stuttgart (1952), Band 8, 4. Auflage, Seiten 122 ff).

Als zu phosgenierende Salze eignen sich vorzugsweise die Hydrochloride oder Ammoniumcarbamate, die durch Sättigung der Diaminlösungen mit gasförmigem Chlorwasserstoff bzw. Kohlendioxid anfallen. Prinzipiell können auch andere Salze, die beispielsweise durch Neutralisation der Diamine mit Protonen abspaltenden Säuren anfallen, phosgeniert werden.

Die Selektivität der Phosgenierungsreaktion ist weitgehend von der Aminkonzentration und vom Phosgenüberschuß abhängig. Vorzugsweise wird das Phosgen in einem hohen molaren Überschuß und das zu phosgenierende Diamin in stark verdünnter Form eingesetzt. Im allgemeinen beträgt der molare Phosgenüberschuß 100 bis 2000 %, vorzugsweise 100 bis 1000 % und die Aminkonzentration, bezogen auf die Gesamtmenge der Amine einerseits und Lösungsmittel andererseits, 0,1 bis 15 Gew.-%, vorzugsweise 5 bis 10 Gew.-%.

Als Lösungsmittel können beliebige inerte organische Flüssigkeiten oder deren Gemische mit einem Siedepunkt von 60 bis 250°C, d.h. Halogenkohlenwasserstoffe, Aromaten, Hydroaromaten sowie deren Chlorverbindungen verwendet werden. Als Beispiele geeigneter Lösungsmittel seien Xylol, Mesitylen, Chlorbenzol, Dichlorbenzol, Trichlorbenzol, Chlornaphthalin und Dichlorethan genannt.

Die Reaktion wird entweder einstufig durch Heißphosgenierung bei Temperaturen von 100 bis 250°C oder zweistufig durch Kalt/Heißphosgenierung bei Temperaturen von -20°C bis 250°C unter Normaldruck durchgeführt.

Bei Verwendung der freien Amine als Ausgangsverbindung (Basenphosgenierung) wird zuerst bei Temperaturen von -20 bis +60°C Ammoniumcarbamidsäurechlorid hergestellt, das dann bei Temperaturen von 20 bis 250°C mit Phosgen zum Diisocyanat weiterreagiert.

Die Reinigung der Verfahrensprodukte erfolgt nach Entphosgenierung durch Abdampfen des Lösungsmittels und anschließende Destillation unter vermindertem Druck.

Die erfindungsgemäßen Verfahrensprodukte, d.h. die neuen erfindungsgemäßen Diisocyanate fallen in hohen Ausbeuten als farblose, niedrigviskose Flüssigkeiten an und stellen wertvolle Aufbaukomponenten bei der Herstellung von Polyurethankunststoffen nach dem Isocyanat-Polyadditionsverfahren dar, Die Stellungs- und/oder Stereoisomerie der neuen Diisocyanate entspricht der Isomerie der bei der Phosgenierung eingesetzten Diamine. Im allgemeinen ist es nicht erforderlich, die beim erfindungsgemäßen Verfahren anfallenden Gemische in einzelne Stellungs- und/ oder Stereoisomere aufzutrennen, da die erfindungsgemäßen Verfahrensprodukte ohne derartige Trennungsoperationen direkt der erfindungsgemäßen Verwendung zugeführt werden können.

Die neuen erfindungsgemäßen Diisocyanate können besonders vorteilhaft zur Herstellung von Polyurethanlacken, Polyurethanelastomeren oder Polyurethanschaumstoffen eingesetzt werden, wobei sie bei den an sich bekannten Verfahren zur Herstellung derartiger Kunststoffe anstelle oder zusammen mit den bislang eingesetzten Polyisocyanaten zum Einsatz gelangen. Besonders vorteilhaft werden die neuen erfindungsgemäßen Diisocyanate bzw. Diisocyanatgemische bei der Herstellung von Polyurethankunststoffen der beispielhaft genannten Art nach dem Prepolymerprinzip verwendet.

Die nachfolgenden Beispiele dienen der weiteren Erläuterung der Erfindung. Alle Prozentangaben beziehen sich, soweit nicht anderslautendes vermerkt, auf Gewichtsprozente. Die Analyse der Isomerenverteilung der Zwischen- und Endprodukte erfolgte gaschromatographisch.

**Beispiel 1**

a) 250 g (1,18 Mol) der bei 198-205°C/0,1 mbar siedenden Hauptfraktion des Polyamingemischs gemäß Beispiel 7c) der EP-A-0 024 665 mit einer Zusammensetzung von 2,4 % 2,2'-Diamino-4-methyldiphenylmethan, 31,3 % 3,2'-Diamino-4-methyldiphenylmethan, 64,5 % 3,4'-Diamino-4-methyldiphenylmethan und 1,8 % undefinierter Diamine und 25 g Ru-Trägerkatalysator (5 % Ru auf $Al_2O_3$) werden in einem 0,7 l Rührautoklaven vorgelegt und nach mehrmaligem Spülen mit Stickstoff und Wasserstoff mit 25 g Ammoniak versetzt. Man erhitzt unter Rühren auf 125°C und bringt im Verlauf von 12,5 Stunden 3,5 Mol Wasserstoff bei 200 bar zur Reaktion. Nach Abtrennung des Katalysators destilliert man das Rohprodukt und unterwirft die Hauptfraktion (Kp: 110-131°C/0,1 mbar) einer Rektifikation.

Dabei wurden 165 g eines bei 127-131°C/0,1 mbar siedenden Diamingemisches gewonnen, das gemäß gaschromatographischem Befund zu 94,3 % aus 1-(1H-Aminocyclohexylmethyl)-3-amino-4-methyl-benzol-Isomeren (die sich zu über 95 % aus solchen Isomeren zusammensetzen, in denen die cycloaliphatisch gebundene Aminogruppe 2- oder 4-ständig ist), zu 2,8 % aus perhydrierten Ausgangsdiaminen und zu 2,9 % aus unhydriertem aromatischem Diamin bestand. Durch die Kernhydrierung bleibt die Verteilung der Stellungsisomeren im wesentlichen erhalten.

b) Man löst 200 g Phosgen in 700 ml Chlorbenzol bei -5 bis 8°C und läßt unter Rühren eine Lösung von 109 g des Diamingemisches aus Beispiel 1a) in 700 ml Chlorbenzol langsam zutropfen. Danach wird unter mäßigem Einleiten von Phosgen auf 120°C erhitzt und weitere 2 Stunden unter gleichen Bedingungen gerührt. Überschüssiges Phosgen wird nun entfernt indem man 1 Stunde am Rückfluß kocht und Stickstoff durch die Lösung bläst. Nach Abdampfen des Lösungsmittels unter vermindertem Druck und Destillation erhält man 112 g eines bei 118 bis 130°C/ 0,05 mbar siedenden 1-(1H-Isocyanato-cyclohexyl-methyl)-3-isocyanato-4-methyl-benzol -Isomerengemisches das einen NCO-Gehalt von 31 %, einen Gehalt von hydrolisierbarem Chlor von 0,04 % und eine Viskosität von 50 mPa.s/25°C besitzt, dessen Isomerenzusammensetzung weitgehend dem Ausgangsprodukt entspricht.

**Beispiel 2**

a) Gemäß EP-A-0 024 665 wird durch Dinitrierung eines Kondensats aus 2-Methyl-benzylchlorid und Benzol und anschließende Hydrierung der so erhaltenen Dinitroverbindung ein Diamingemisch folgender Zusammensetzung hergestellt:

1 % 6, 2'-Diamino-2-methyl-diphenylmethan,

14,5 % eines Isomerengemisches aus 3, 2'- und 5, 2'-Diamino-2-methyldiphenylmethan,

0,9 % 6, 3'-Diamino-2-methyl-diphenylmethan,

7,9 % eines Isomerengemischs aus 4, 2'-, 6, 4'-Diamino-2-methyl-diphenylmethan,

75 % eines Isomerengemischs, welches zu mehr als 80 % aus 3,4'- und 5,4'-Diamino-2-methyl-diphenylmethan und zum Rest aus anderen Diamino-2-methyl-diphenylmethanen besteht und

0,7 % undefinierte Polyamine.

250 g (1,18 Mol) dieses Diamingemischs werden innerhalb von 22 Stunden bei 125°C und 200 bar mit Ru-Trägerkatalysator analog Beispiel 1 hydriert.

Die bei 100 bis 146°C/0,1 mbar siedende Hauptfraktion bestand laut gaschromatographischem Befund aus 41,0 g (15,5 % der Theorie) des, dem Ausgangsdiamin entsprechenden, perhydriertem Diamin, 190,9 g (74,2 % der Theorie) aus, dem Ausgangsdiamin entsprechenden, (1H-Aminocyclohexyl-methyl)-2-methyl-anilinen und zu 23,8 g (9,5 % der Theorie) aus unverändertem Ausgangsmaterial. Als Destillationsrückstand fallen 2,1 g (0,8 % der Theorie) an.

Eine nochmalige fraktionierte Destillation bei 138 bis 142°C/0,1 mbar ergab 171,1 g (1H-Aminocyclohexyl-methyl)-methylaniline, die bezüglich der Zusammensetzung dem Ausgangsprodukt entsprechen und die im Gemisch mit 4,6 % perhydriertem Diamin und 2,0 % Ausgangsprodukt vorliegen.

b) Davon löst man 109 g in 700 ml Chlorbenzol und läßt diese Lösung unter intensivem Rühren bei 0 bis 8°C in eine Lösung von 200 g Phosgen in 700 ml Chlorbenzol zutropfen. Es bildet sich ein zäher Niederschlag der sich nach langsamem Aufheizen unter Einleiten von Phosgen allmählich auflöst. Man kocht das bei 105°C klar werdende Reaktionsgemisch noch 2 Stunden am Rückfluß, stoppt die Phosgenzufuhr und entphosgeniert, indem man Stickstoff einleitet. Nach Abdampfen des Lösungsmittels unter vermindertem Druck reinigt man das Diisocyanatgemisch durch Destillation. Dabei wurden 104 g Diisocyanat mit einem Siedepunkt von 125 bis 129°C/0,04 mbar, einem NCO-Gehalt von 31,0 %, einem Gehalt an hydrolisierbarem Chlor von 0,03 % und einer Viskosität von 70 mPa.s/25°C gewonnen, das folgende Zusammensetzung besitzt:

1,6 % 2-(6-Isocyanato-2-methylbenzyl)-cyclohexyl-isocyanat,

9,1 % Gemisch aus 4-(4-Isocyanato-2-methylbenzyl)-, 4-(6-Isocyanato-2-methylbenzyl)- und 3-(6-Isocyanato-2-methylbenzyl)-cyclohexylisocyanat,

12,0 % Gemisch aus 2-(3-Isocyanato-2vmethylbenzyl)-und 2-(5-Isocyanato-2-methylbenzyl)-cyclohexylisocyanat,

77,3 % Gemisch, das zu mindestens 80 % aus 4-(3-Isocyanato-2-methylbenzyl)- und 4-(5-Isocyanato-2-methylbenzyl)-cyclohexylisocyanat.

**Beispiel 3**

a) In einem 0,7 l Rührautoklaven wurden analog Beispiel 1a) 250 g (1,18 Mol) 5, 4'-Diamino-2-methyl-diphenylmethan in Gegenwart von 25 g Ru-Trägerkatalysator (5 % Ru auf $Al_2O_3$ und 25 g Ammoniak bei 125° C und 200 bar 11,5 Stunden hydriert. Nach Flash-Destillation bei 120 bis 170° C (0,2 bis 0,5 mbar) und anschließender Feindestillation wurden 165 g Diamin gewonnen, das einen Siedepunkt von 130 bis 135° C/ 0,05 mbar aufweist und laut gaschromatographischer Analyse aus 5 % 5,4'-Diamino-2-methyl-dicyclohexyl-methan, 94,4 % 4-(5-Amino-2-methylbenzyl)-cyclohexyl-amin und 0,6 % 5, 4'-Diamino-2-methyl-diphenylmethan besteht.

b) Man löst bei -5 bis 8° C 200 g Phosgen in 700 ml Chlorbenzol und läßt unter Rühren eine Lösung von 109 g des Diamingemisches aus Beispiel 3a) in 700 ml Chlorbenzol zutropfen. Dabei scheidet sich ein zäher Feststoff aus und das Gemisch erwärmt sich auf ca. 30° C. Man erhöht die Temperatur langsam unter gleichzeitigem Einleiten von 100 g/h Phosgen bis bei ca, 110° C der Feststoff in Lösung geht. Danach rührt man noch weitere 3 Stunden unter Phosgenzuführung bei 130° C, entphosgeniert und destilliert das Rohprodukt. Dabei wurden 109 g 4-(5-Isocyanato-2-methylbenzyl)-cyclohexylisocyanat mit einem Siedepunkt von 125 bis 127° C/0,1 mbar, einem NCO-Gehalt von 31,0 %, einem Gehalt an hydrolisierbarem Chlor von 0,02 % und einer Viskosität von 75 mPa.s/ 25° C gewonnen.

**Beispiel 4**

a) Ein 0,7 l Rührautoklav wird mit 250 g (1,18 Mol) 3,4'-Diamino-4-methyldiphenylmethan und 25 g $Ru/Al_2O_3$ (Ru-Gehalt 5 Gew.-%) beschickt. Nach mehrmaligem Spülen mit Stickstoff und Wasserstoff werden über eine Lewa-Pumpe 25 g flüssiges Ammoniak zudosiert, der Autoklav unter Rühren auf 125° C erwärmt und bei 200 bar hydriert, bis nach 16 Stunden ca. 3,5 Mol Wasserstoff absorbiert sind. Man stellt den Rührer ab, läßt abkühlen, entspannt den Autoklaven und nimmt das Produkt in Methanol auf. Nun filtriert man den Katalysator ab, wäscht mit Methanol nach und vereinigt die Methanollösungen. Nach Abdampfen des Lösungsmittels und Flash-Destillation bei 0,1 bar und 100 bis 150° C erhält man 254 g eines Gemisches, das laut gaschromatographischer Analyse aus 219,5 g (85,3 % d.Th.) 4-(3-Amino-4-methylbenzyl)-cyclohexylamin und 34,0 g (12,9 % d.Th.) 3,4'-Diamino-4-methyl-dicyclohexylmethan besteht. Durch zusätzliche Destillation über eine Vigreux-Kolonne bei 145-146° C/0,1 mbar konnten 150 g 4-(3-Amino-4-methylbenzyl)-cyclohexylamin isoliert werden. $C_{14}H_{22}N_2$;

ber: C 77,06 H 10,10 N 12,84

gef: C 76,6 H 10,1 N 12,8

(alle Zahlenwerte in %).

b) Man löst 95 g Phosgen bei -5° C in 350 ml wasserfreiem Chlorbenzol und läßt unter intensivem Rühren eine Lösung von 52 g (0,238 Mol) 4-(3-Amino-4-methylbenzyl)-cyclohexylamin in 350 ml wasserfreiem Chlorbenzol zutropfen. Die entstandene Suspension wird unter Einleiten von Phosgen auf 65° C erwärmt bis sich der Feststoff löst und anschließend 3 Stunden bei 120° C nachphosgeniert. Nach 2-stündigem Entphosgenieren wird das Lösungsmittel unter vermindertem Druck abdestilliert und das Diisocyanat bei 133 bis 135° C/0,1 mbar destillativ gereinigt. Dabei werden 58,3 g (90,7 % d.Th.) 4-(3-Isocyanato-4-me-thylbenzyl)-cyclohexylisocyanat gewonnen, das einen NCO-Gehalt von 31,0 %, einen Gehalt an hydrolysierbarem Chlor von weniger als 0,01 % und eine Viskosität von 50 mPa.s/25° C aufweist.

**Beispiel 5**

a) In einem 0,7 l Rührautoklaven werden 25 g Ruthenium-Aluminiumoxid-Trägerkatalysator und 250 g (1,106 Mol) eines gemäß EP-A 46 556 (Beispiel 5) hergestellten Diamino-ethyl-diphenylmethan-Isomerengemisches vorgelegt, das folgende Zusammensetzung besitzt:

1,9 % Gemisch aus 2,2'-Diamino-4- und 2, 2'-Diamino-6-ethyldiphenylmethan,

11,1 % 4, 2'-Diamino-2-ethyldiphenylmethan,

20,1 % Gemisch aus 3,2-Diamino-2-, 3,2'-Diamino-4-und 3,2'-Diamino-6-ethyldiphenylmethan,

66,1 % Gemisch, das zu mehr als 80 % aus 3,4'-Di-amino-2-, 3,4'-Diamino-4- und 3,4'-Diamino-6-ethyldiphenylmethan besteht und bis zu 20 % andere Diamino-ethyldiphenylmethane enthält,

0,8 % unbekannte Triamine.

Nach mehrmaligem Spülen mit Stickstoff und Wasserstoff dosiert man 25 g Ammoniak zu und hydriert bei 130° C und 200 bar bis 3,3 Mol Wasserstoff absorbiert sind. Danach läßt man abkühlen, entspannt, löst das Produkt in Methanol auf und saugt den Katalysator ab. Nach Abdestillieren des Lösungsmittels wird das Rohprodukt zweimal bei 0,05 mbar destilliert. Man erhält 180 g eines bei 125 bis 136° C/0,05 mbar siedenden Diamingemisches, das zu 93,6 % aus 1H-Amino-ethylbenzyl-cyclohexylamin mit einer dem Ausgangsdiamin entsprechenden Isomerenzusammensetzung, 2,9 % aus pherhydriertem und 3,5 % aus unhydriertem Ausgangsprodukt besteht.

b) Bei -10 bis -5° C versetzt man eine Lösung von 150 g Phosgen in 650 ml Chlorbenzol mit einer Lösung von

100 g des Diamingemisches aus Beispiel 5a) in 650 ml Chlorbenzol und erhitzt das Reaktionsgemisch unter Einleiten von Phosgen auf Rückflußtemperatur. Nach weiterem 90-minütigem Rühren unter gleichen Bedingungen wird entphosgeniert, das Produkt eingeengt und zweimal destilliert. Als Ausbeute erhält man 104 g eines bei 140 bis 144°C/0,1 mbar siedenden Diisocyanats, das zu 99,5 % aus verschiedenen, dem Ausgangsprodukt entsprechenden, 1H-Isocyanato-ethylbenzyl-cyclohexylisocyanat-Isomeren besteht. Das Produkt ist durch folgende Daten charakterisiert:

NCO-Wert: 29,6 %
Gehalt an hydrolysierbarem Chlor: < 0,01 %
Viskosität: 52 mPa.s/25°C.

**Patentansprüche**

1) Gegebenenfalls Isomerengemische darstellende und gegebenenfalls im Gemisch mit untergeordneten Mengen an den entsprechenden perhydrierten Diisocyanaten und/oder im Gemisch mit untergeordneten Mengen an den entsprechenden unhydrierten aromatischen Diisocyanaten vorliegende Diisocyanate der Formel

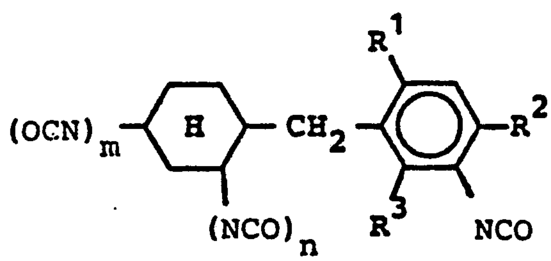

in welcher
$R^1$, $R^2$ und $R^3$ für gleiche oder verschiedene Reste stehen und Wasserstoff oder (gegebenenfalls verzweigte) Alkylgruppen mit 1 bis 12 Kohlenstoffatomen bedeuten, mit der Einschränkung, daß zwei der Reste $R^1$, $R^2$ und $R^3$ für Wasserstoff stehen und
m und n jeweils für 0 oder 1 stehen, mit der Maßgabe, daß die Summe $m + n$ den Wert 1 ergibt, wobei im Falle von m oder $n = 0$ die freie Valenz durch Wasserstoff abgesättigt ist.

2) Diisocyanate gemäß Anspruch 1 der allgemeinen Formel

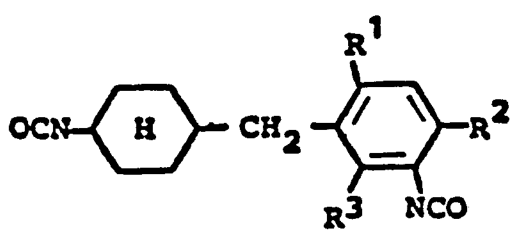

oder Diisocyanatgemische gemäß Anspruch 1, deren Hauptkomponente dieser Formel entspricht, oder deren Hauptkomponenten dieser Formel entsprechen, dadurch gekennzeichnet, daß
einer der Reste $R^1$, $R^2$ oder $R^3$ für einen Alkylrest mit 1 bis 4 Kohlenstoffatomen, insbesondere einen Methylrest und die verbleibenden der genannten Reste für Wasserstoff stehen.

3. Diisocyanate gemäß Anspruch 1 der allgemeinen Formel

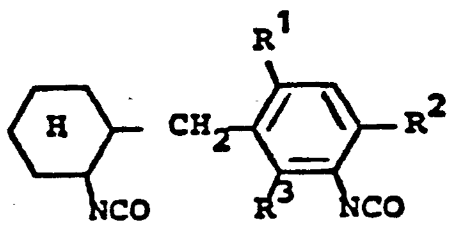

oder Diisocyanatgemische gemäß Anspruch 1, deren Hauptkomponente dieser Formel entspricht, oder deren Hauptkomponenten dieser Formel entsprechen, dadurch gekennzeichnet, daß

einer der Reste $R^1$, $R^2$ oder $R^3$ für einen Alkylrest mit 1 bis 4 Kohlenstoffatomen, insbesondere einen Methylrest und die verbleibenden der genannten Reste für Wasserstoff stehen.

4) Verfahren zur Herstellung von Diisocyanaten oder Diisocyanatgemischen gemäß Anspruch 1, dadurch gekennzeichnet, daß man, gegebenenfalls Isomerengemische darstellende und gegebenenfalls im Gemisch mit untergeordneten Mengen an den entsprechenden perhydrierten Diaminen und/oder gegebenenfalls im Gemisch mit untergeordneten Mengen an den entsprechenden unhydrierten aromatischen Diaminen vorliegende Diamine der Formel

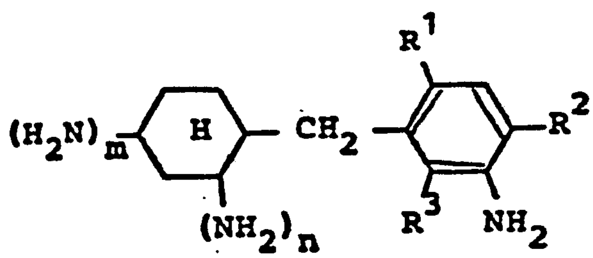

in welcher

$R^1$, $R^2$, $R^3$, m und n die in Anspruch 1 genannte Bedeutung haben,

bei -20°C bis +250°C phosgeniert.

5) Verwendung der Diisocyanate bzw. Diisocyanatgemische gemäß Anspruch 1 bis 3 als Aufbaukomponente bei der Herstellung von Polyurethankunststoffen nach dem Isocyanat-Polyadditionsverfahren.

**Claims**

1. Diisocyanates which are optionally in the form of isomer mixtures and are optionally mixed with minor quantities of the corresponding perhydrogenated diisocyanates and/or with minor quantities of the corresponding unhydrogenated aromatic diisocyanates and which have the formula

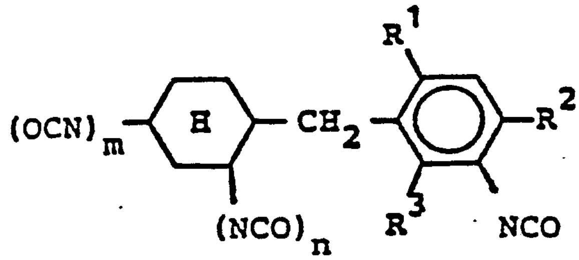

in which
$R^1$, $R^2$ and $R^3$ represent identical or different radicals and denote hydrogen or (optionally branched) alkyl groups with 1 to 12 carbon atoms, with the limitation that two of the radicals $R^1$, $R^2$ and $R^3$ represent hydrogen and m and n each represent 0 or 1, with the proviso that the sum of m + n is 1, and if m or n = 0 the free valency is saturated by hydrogen.

2. Diisocyanates according to Claim 1 of the general formula

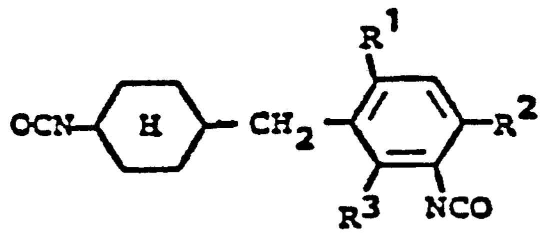

or diisocyanate mixtures according to Claim 1, the main component of which corresponds to this formula, or the main components of which correspond to this formula, characterised in that one of the radicals $R^1$, $R^2$ or $R^3$ represents an alkyl radical with 1 to 4 carbon atoms, in particular a methyl radical, and the remaining radicals of those mentioned represent hydrogen.

3. Diisocyanates according to Claim 1 of the general formula

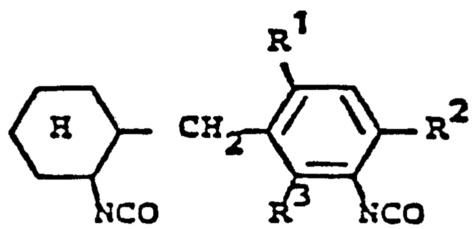

or diisocyanate mixtures according to Claim 1, the main component of which corresponds to this formula, or the main components of which correspond to this formula, characterised in that one of the radicals $R^1$, $R^2$ or $R^3$ represents an alkyl radical with 1 to 4 carbon atoms, in particular a methyl radical and the remaiming radicals of those mentioned represent hydrogen.

4. Process for the preparation of diisocyanates or diisocyanate mixtures according to Claim 1, characterised in that diamines which are optionally in the form of isomer mixtures and are optionally mixed with minor quantities of the corresponding perhydrogenated diamines and/or with minor quantities of the corresponding unhydrogenated aromatic diamines and which have the formula

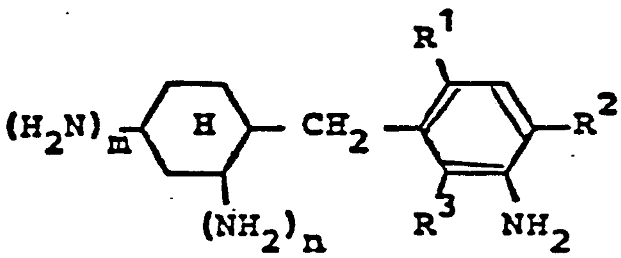

in which

$R^1$, $R^2$, $R^3$, m and n have the meaning given in Claim 1, are phosgemated at -20°C to +250°C.

5. Use of the diisocyanates or diisocyanate mixtures according to Claim 1 to 3 as a component in the production of polyurethane plastics by the isocyanate polyaddition process.

**Revendications**

1. Diisocyanates, constitués éventuellement de mélanges d'isomères et éventuellement mélangés avec des proportions mineures des diisocyanates perhydrogénés correspondants et/ou des proportions mineures des diisocyanates aromatiques non hydrogénés correspondants, qui répondent à la formule

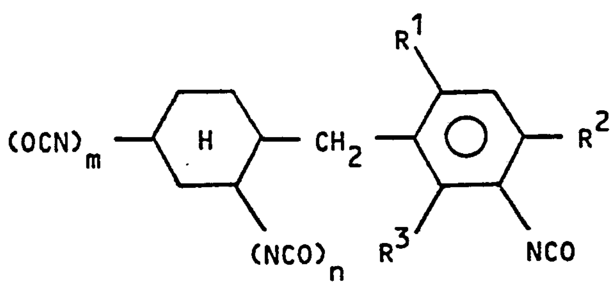

dans laquelle

$R^1$, $R^2$ et $R^3$, ayant des significations identiques ou differentes, représentent chacun l'hydrogène ou un groupe alkyle en $C_1$-$C_{12}$, éventuellement ramifié, avec la limitation que deux des symboles $R^1$, $R^2$ et $R^3$ doivent représenter l'hydrogène, et m et n sont chacun égaux à 0 ou 1, avec la condition que la somme m + n est égale à 1, la valence libre dans le cas où m ou n = 0 étant saturée par l'hydrogène.

2. Diisocyanates selon la revendication 1, de formule générale

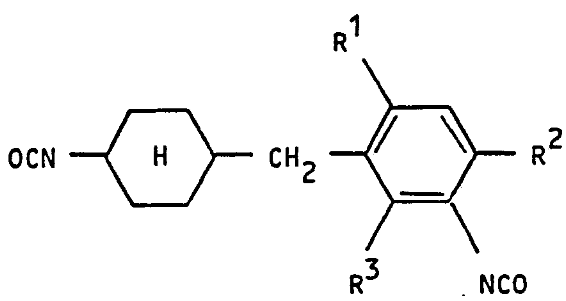

ou mélanges de diisocyanates selon la revendication 1, dont le constituant principal répond à cette formule ou dont les constituants principaux repondent à cette formule, caractérisés en ce que l'un des symboles $R^1$, $R^2$ ou $R^3$ représente un groupe alkyle en $C_1$-$C_4$, en particulier un groupe méthyle, et les autres représentent l'hydrogène.

3. Diisocyanates selon la revendication 1, de formule générale

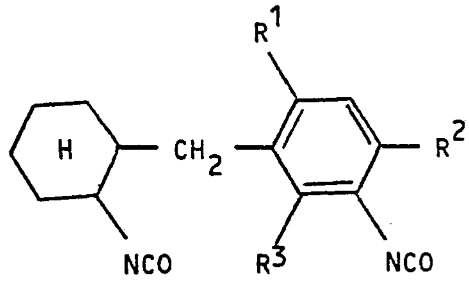

ou mélanges de diisocyanates selon la revendication 1, dont le constituant principal répond à cette formule ou dont les constituants principaux repondent à cette formule, caractérisés en ce que l'un des symboles $R^1$, $R^2$ ou $R^3$ représente un groupe alkyle en $C_1$-$C_4$, en particulier un groupe méthyle, et les autres l'hydrogène.

4. Procédé de préparation des diisocyanates ou mélanges de diisocyanates selon la revendication 1, caractérisé en ce que l'on soumet à phosgénation à des températures de -20 à +250°C des diamines, consistant éventuellement en mélanges d'isomères et éventuellement mélangées avec des proportions mineures des diamines perhydrogénées correspondantes et/ou éventuellement mélangées avec des proportions mineures des diamines aromatiques non hydrogénées correspondantes, qui répondent à la formule

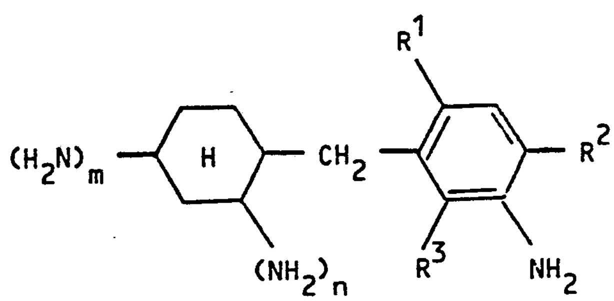

dans laquelle
$R^1$, $R^2$, $R^3$, m et n ont les significations indiquées dans la revendication 1.

5. Utilisation des diisocyanates ou mélanges de diisocyanates selon les revendications 1 à 3 en tant que composants de synthèse à la préparation de résines synthétiques de polyuréthannes par le procédé de polyaddition des isocyanates.